**Europäisches Patentamt**

**European Patent Office**

**Office européen des brevets**

(19)

(11) Veröffentlichungsnummer : **0 018 541 B2**

(12) : **NEUE EUROPÄISCHE PATENTSCHRIFT**

(45) Veröffentlichungstag der neuen Patentschrift :
**10.04.91 Patentblatt 91/15**

(51) Int. Cl.$^5$ : **C07C 303/06, C07C 309/30**

(21) Anmeldenummer : **80102014.0**

(22) Anmeldetag : **15.04.80**

(54) Verfahren zur Herstellung von 4-Nitrotoluol-2-sulfonsäure.

Die Akte enthält technische Angaben, die nach dem Eingang der Anmeldung eingereicht wurden und die nicht in dieser Patentschrift enthalten sind.

(30) Priorität : **26.04.79 DE 2916912**

(43) Veröffentlichungstag der Anmeldung :
**12.11.80 Patentblatt 80/23**

(45) Bekanntmachung des Hinweises auf die Patenterteilung :
**13.07.83 Patentblatt 83/28**

(45) Bekanntmachung des Hinweises auf die Entscheidung über den Einspruch :
**10.04.91 Patentblatt 91/15**

(84) Benannte Vertragsstaaten :
**CH DE FR GB LI**

(56) Entgegenhaltungen :
EP-B- 0 083 555
CH-A- 478 772
DE-A- 1 643 607
DE-A- 2 419 455
FR-A- 1 544 998
FR-A- 1 555 394
FR-A- 2 164 182
GB-A- 1 164 752
JP-A-55 004 357

(56) Entgegenhaltungen :
US-A- 3 840 591
Ullmanns Encyklopädie der technischen Chemie, 4. Auflage, Bd. 17, Verlag Chemie, Weinheim, New York, 1979, S. 404, 405
Fleury, R., "Beitrag zur Kenntnis der Sulfonierung aromatischer Verbindungen mit Schwefeltrioxyd", Abhandlung zur Erlangung der Würde eines Doktors der Eidgenössischen Technischen Hochschule Zürich, 1966, Dissertation Nr.3857,S. 22,29 und 47
Guyer, P. et al., Chimia, 22 (1968), 40 -43
Rogenass, W., et al., "Reactor Engineering for Inherent Safety", I. Chem. E. Symposium Series No. 87, 369 - 376
Chimia 22, 40-43, 1968
Ullmanns Encyklopädie der technischen Chemie, 3. Aufl., Bd.15,1964,S.466-67
Institution of Chemical Engineers, Symposium Series 1984, Nr.87,S. 371-73 (C.A.102, 97 567 a)

(73) Patentinhaber : **BAYER AG**
**W-5090 Leverkusen 1 Bayerwerk (DE)**

(72) Erfinder : **Goldschmitt, Ernst, Dr.**
**Andreas-Gryphius-Strasse 9**
**W-5000 Köln 80 (DE)**
Erfinder : **Schnegg, Peter, Dr.**
**Heidberger Strasse 9**
**W-5068 Odenthal (DE)**
Erfinder : **Blank, Heinz Ulrich, Dr.**
**Am Geusfelde 35**
**W-5068 Odenthal (DE)**

**EP 0 018 541 B2**

## Beschreibung

Verfahren zur Herstellung von
4-Nitrotoluol-2-sulfonsäure

Die Erfindung betrifft ein Verfahren zur Herstellung von 4-Nitrotoluol-2-sulfonsäure durch Umsetzung von geschmolzenem 4-Nitrotoluol mit gasförmigem Schwefeltrioxid.

Est ist bekannt (DE–OS 2 419 455), p-Nitrotoluol-2-sulfonsäure durch Umsetzung von geschmolzenem p-Nitrotoluol mit einem Gemisch aus höchstens 50 Vol.-% gasförmigen Schwefeltrioxid und einem Inertgas bei einer Temperatur von 90 bis 150°C herzustellen.

Dabei wird das Reaktionsgemisch als eine schwarze, teerartige Aufschlämmung gegen Ende der Schwefeltrioxidzugabe erhalten, aus der nach Hydrolyse des p-Nitrotoluol-2-sulfonsäure-anhydrids mit Wasser eine Wäßrige p-Nitrotoluol-2-sulfonsäurelösung entsteht, die vor oder nach Abtrennung von wasserunlöslichen Nebenprodukten mit Aktivkohle behandelt wird, um so gelöste, gefärbte Verbindungen und gelöstes, nicht umgesetztes p-Nitrotoluol abzutrennen.

Die in der DE–OS 2 419 455 beschriebenen Verfahrensweise hat den Nachteil, daß mit Inertgas-verdünntem Schwefeltrioxid gearbeitet wird. Dadurch wird ein erheblicher technischer Aufwand zur Vorreinigung des Inertgases notwendig, z.B. zur Trocknung des Inertgases bzw. zur Nachreinigung, wie der Abtrennung von im inertgasstrom mit- gerissenem, flüchtigem p-Nitrotoluol und/oder Schwefeltrioxid. Weiterhin ist bei dieser Arbeitsweise eine spezielle Vorrichtung zur Ableitung des inertgasstromes aus dem Reaktionsgefäß erforderlich. Trotz dieses erheblichen technischen Aufwandes werden nur etwa 95% Ausbeute an p-Nitrotoluol-2-sulfonsäure erzielt, wobei das Produkt teerartige Verunreinigungen enthält.

Weiterhin ist aus der DE–OS 2 353 918 und der DE–OS 2 354 097 ein Verfahren zur Darstellung von aromatischen Sulfonsäuren durch Behandlung von aromatischen Kohlenwasserstoffen mit gasförmigem SO₃ bekannt, bei dem die Reaktion unter ständig aufrechterhaltenem größem Überschuß von aromatischem Kohlenwasserstoff als Wärmeträgermedium unter Rückflußbedingungen stattfindet, wobei gemäß der DE–OS 2 354 097 als Wärmeträgermedium eine inerte, die aromatische Substanz und die aus ihr gebildete Sulfonsäure lösende Verbindung eingesetzt wird. Die Reaktionstemperatur, die über den Druck geregelt wird, liegt zwischen 20 und 100°C und stimmt mit der Rückflußtemperatur überein.

Nach dem oben geschilderten Verfahren ist aus physikalischen Gegebenheiten im Falle der Sulfonierung von p-Nitrotoluol (Siedepunkt 105°C/12 mbar) das lösungsmittelfreie Verfahren technisch nur mit erheblichem Aufwand durchführbar. Auch die vorgeschlagene Verwendung von Lösungsmitteln bedeutet einen zusätzlichen technischen Aufwand, sofern überhaupt im fraglichen Temperaturbereich gegen SO₃ genügend stabile organische Lösungsmittel zur Verfügung stehen.

Weiterhin ist aus FR 1 555 394, Beispiel 2, die Herstellung von p-Nitrotoluolsulfonsäure bekannt, wobei dem geschmolzenen p-Nitrotoluol (1 Mol) bei 80-90°C gasförmiges SO₃ (1,1 Mol) zugeführt wird. Anschließend wird die Temperatur auf 115-120°C gesteigert und 8 Stunden beibehalten. Die heiße Schmelze ergibt nach Ablassen in Wasser eine dunkelbraune Lösung der p-Nitrotoluolsulfonsäure. Entsprechend CH 478 772, Spalte 2, Absatz 3 kann die Sulfonierung auch ohne Lösungsmittel durch Einleiten von gasförmigem, stark mit indifferenten Gasen, wie Stickstoff, verdünntem Schwefeltrioxid oder oxidierten Restgasen z.B. aus einer Kontakt-Schwefelsäureanlage in den flüssigen oder geschmolzenen Nitroaromaten durchgeführt werden.

Es wurde nun ein Verfahren zur Herstellung von 4-Nitrotoluol-2-sulfonsäure durch Umsetzung von geschmolzenem 4-Nitrotoluol mit gasförmigen Schwefeltrioxid bei erhöhter Temperatur, Nachreaktion des Reaktionsgemisches und anschließendes Zusammengeben mit Wasser gefunden, das dadurch gekennzeichnet ist, daß man in des geschmolzene 4-Nitrotoluol bei einer Temperatur im Bereich von 90-150°C unverdünntes gasförmiges Schwefeltrioxid, das technische Verunreinigungen enthalten kann, bei Drücken oberhalb des Dampfdruckes des 4-Nitrotoluols im Molverhältnis von SO₃ zu 4-Nitrotoluol von 0,7 : 1 bis 1,3 : 1 einleitet und das Sulfiergemisch nach Erreichen eines Umsatzes von mindestens 85% der im Unterschuß eingesetzten Reaktions-Komponente mit Wasser zusamengibt.

Das dabei erhaltene Reaktionsgemisch kann einer weiteren Reinigung unterworfen werden.

Bei dem erfindungsgemäßen Verfahren wird das Sulfiergemisch nach Erreichen eines Umsatztes von mindestens 85% der im Unterschuß eingesetzten Reaktionskomponente mit einer solchen Menge an Wasser zusammengegeben, daß nach Hydrolyse der im Gemisch vorhandenen Sulfonsäureanhydride bei etwa 80 bis 100°C in etwa 30 bis 60 Minuten eine 4-Nitrotoluol-2-sulfonsäure-Lösung der für die Weiterverarbeitung geeigneten Konzentration, beispielsweise von ca. 20 bis 70 Gew.-%, entsteht, die absorptiv oder extraktiv gereinigt werden kann. Die Lösung wird dabei vorzugsweise, gegebenenfalls nach Filtration des schwerlöslichen Sulfons, mit einem mit Wasser nicht beliebig mischbaren organischen Lösungsmittel extrahiert. Nach Abtrennen der Restmengen des zur Extraktion verwendeten Lösungsmittels erhält man eine ca. 40%ige 4-Nitrotoluol-2-sulfonsäure-Reinlösung von aus gezeichneter Qualität wie aus folgenden Daten zu entnehmen ist :

Summe aller organischen Verunreinigungen : 1 ppm
Farbzahl einer 40%igen Lösung : 4 bis 6
(Gardner Färbestandard ; vgl. DE–OS 2 419 455).

Das Wasser, das dem Reaktionsgemisch nach Erreichen eines Umsatzes von mindestens 85% der im Unterschuß eingesetzten Reaktionskomponente zugesetzt wird, kann auch im Form wäßriger Alkalihydroxid-Lösungen und/oder vorgereinigter oder, vorzugsweise, nicht vorgereinigter saurer, neutraler oder alkalischer wäßriger Lösungen, die 4-Nitrotoluolsulfonsäure bereits in einer solchen Menge gelöst enthalten, daß sich zusammen mit der 4-Nitrotoluosulfonsäure aus dem Sulflergemisch eine Konzentration an 4-Nitrotoluolsulfonsäure von ca. 20 bis 70 Gew.-% einstellt, eingesetzt werden.

Die Ausbeute an 4-Nitrotoluol-2-sulfonsäure lieght bei etwa 98 bis 99,5% der Theorie (bezogen auf umgesetztes 4-Nitrotoluol).

Das obige Ergebnis ist um so überraschender, als nach dem Stand der Technik angenommen werden mußte, daß 4-Nitrotoluol mit Schwefeltrioxid nur bei Anwendung spezieller Maßnahamen, wie inertgasverdünnung, Rückflußbedingungen, Verwendung von Lösungsmitteln bzw. Verdünnungsmitteln, großem Überschuß an zu sulfonierenden aromatischen Kohlenwasserstoffen mit guter Ausbeute zu einem Produkt befriedigender Qualität umgesetzt werden kann.

Wie zuvor beschrieben, sind Reaktionstemperaturen im Bereich von 90 bis 150°C für die Sulfierung geeignet, wobei Reaktionstemperaturen von 90 bis 130°C und insbesondere von 90 bis 125°C vorteilhaft sind. Besonders bevorzugt ist das Temperaturbereich von 100 bis 120°C.

Erfindungsgemäß arbeitet man bei Drücken oberhalb des Dampfdruckes des 4-Nitrotoluols.

Beim Einleiten des gasförmigen Schwefeltrioxids in der Reaktionsgemisch ist ein gewisser Überdruck zur Überwindung des hydrostatischen Drucks des 4-Nitrotoluols erforderlich, wobei zu beachten ist, daß keine kondensation des Schwefeltrioxides erfolgt.

Das erfindungsgemäße Verfahren kann durchgeführt werden, indem man gasförmiges $SO_3$ mit geschmolzenem 4-Nitrotoluol im Molverhältnis von etwa 0,7 : 1 bis 1,3 : 1, vorzugsweise 0,9 : 1 bis 1,2 : 1, im Temperaturbereich von etwa 80 bis 130°C intensiv vermischt und während des Mischens durch Kühlung die gewählte Reaktionstemperatur einhält. Einige Minuten nach dem Mischen, spätestens nach etwa 10 Stunden, ist unter den Reaktions-bedingungen ein Umsatz vom mindestens 85% der Theorie, bezogen auf die im Unterschuß eingesetzte Reaktionskomponente, erreicht. Der Umsatz der im Unterschuß eingesetzten Komponente beträgt vorzugsweise mehr als 90% der Theorie.

Nach Erreichen des gewünschten Umsatzes wird das Reaktionsgemisch beispielsweise mit 100 bis 500 ml Wasser pro Mol eingesetztem 4-Nitrotoluol versetzt und bei erhöhter Temperatur bei ca. 80 bis 100°C im allgemeinen bis etwa 60 Minuten lang gerührt. Man erhält eine ca. 20 bis 70%ige, fahlgelbe bis rötliche 4-Nitrotoluol-2-sulfonsäure-Rohlösung, in der das nicht umgesetzte 4-Nitrotoluol gelöst bzw. emulgiert ist und in der gegebenenfalls geringe Mengen an kristallinem Sulfon suspendiert sind. Die Ausbeute an 4-Nitrotoluol-2-sulfonsäure liegt bei ≥ 98% der Theorie (bezogen auf umgesetztes 4-Nitrotoluol).

Aus der wäßrigen 4-Nitrotoluol-2-sulfonsäure-Rohlösung kann, gegebenefals nach Filtration und/oder Phasentrennung, durch Einegen und/oder Kühlen und/oder durch Einstellung einer geeigneten $(H_3O)^+$-Ionenkonzentration, 4-Nitrotoluol-2-sulfonsäure kristallin ausgefällt und isoliert werden.

Vorzugsweise wird aus der 4-Nitrotoluol-2-sulfonsäure-Rohlösung, gegebenenfalls nach Filtration des ungelösten Sulfons, eine Reinlösung dadurch hergestellt, daß die Suspension bzw. Lösung mit einem geeigneten Lösungsmittel, gegebenenfalls nach Abtrennung von überschüssigem 4-Nitrotoluol, gegebenenfalls bei erhöhter Temperatur, extrahiert wird.

Als Lösungsmittel für die Extraktion kommen in Frage mit Wasser nicht beliebig mischbare, organische Lösungsmittel, beispielsweise chlorierte aliphatische Kohlenwasserstoffe mit 1 bis 5 Kohlenstoffatomen, bevorzugt 1 bis 3 Kohlenstoffatomen wie Methylenchlorid, Chloroform, Tetrachlorkohlenstoff, Dichloräthan und/oder aromatische Kohlenwasserstoffe mit 6 bis 18 Kohlenstoffatomen, bevorzugt 6 bis 12 Kohlenstoffatomen, wie Chlorbenzol, Dichlorbenzol, Benzol, Toluol, o-, m-, p-Xylol, Ethylbenzol, Cumol, Tetralin, Chlornaphthalin, Methylnaphthalin.

Bevorzugt werden Methylenchlorid und/oder Alkylbenzole, im besonderen Toluol, als Lösungsmittel für die Extraktion verwendet.

Mit dem organischen Lösungsmittel wird im Verhältnis von etwa 1 : 25 bis etwa 2 : 1 Gew.- Teilen zur Rohlösung, vorzugsweise im Verhältnis 1 : 10 bis 1 : 20, ein oder mehrere Male extrahiert.

Gegebenenfalls wird nach der letzten Extraktion der Restanteil des in der wäßrigen Phase gelösten organischen Lösungsmitells durch Andestillieren entfernt.

Auf diese Weise erzeugt man gewöhnlich eine ca. 20 bis 70%ige wäßrige 4-Nitrotoluol-2-sulfonsäure-Lösung mit der gewünschten hochen Reinheit. Die Konzentration an freier Schwefelsäure beträgt in der 4-Nitrotoluol-2-sulfonsäure-Lösung allgemeinen weniger als 10 Mol-% und liegt bevorzugt bei etwa 3 bis 7 Mol-% (bezogen auf 4-Nitrotoluol-2-sulfonsäure).

Die Extraktion kann diskontinuierlich oder kontinuierlich durchgeführt werden.

Das gegebenenfalls durch Phasentrennung aus der wäßrigen Lösung abgetrennte und/oder beispielsweise extraktiv zurückgewonnene, nicht umgesetzte 4-Nitrotoluol kann erneut in die Sulfierung eingesetzt werden.

In einer weiteren Ausführungsform des erfindungsgemäßen Verfahrens wird SO$_3$ im Überschuß von bis zu 20%, vorzugsweise bis zu 15%, bezogen auf eingesetztes 4-Nitrotoluol, eingesetzt und das Reaktionsgemisch nach Erreichen eines nahezu quantitativen 4-Nitrotoluol-Umsatzes mit Wasser zusammengegeben. Die Restmengen an 4-Nitrotoluol werden, gegebenenfalls nach dem Abfiltrieren von als Nebenprodukt entstandenem 5,5'-Dinitro-2,2'-ditolylsulfon, z.B. durch Extraktion abgetrennt.

In einer bevorzugten Ausführungsform des erfindungsgemäßen Verfahrens werden SO$_3$ und 4-Nitrotoluol im molaren Verhältnis von 0,9 : 1 bis 1,2 : 1 eingesetzt und die Umsetzung durch Versetzen mit Wasser dann unterbrochen, wenn die im Unterschuß eingesetzte Komponente sich zu mehr als 90% umgesetzt hat. Nach Hydrolyse das Sulfiergemisches wird der Anteil des nicht umgesetzten 4-Nitrotoluols gegebenenfalls nach Abtrennung des im Reaktionsverlauf gebildeten 5,5'-Dinitro-2,2'-ditolylsulfons in die Reaktion zurückgeführt. Die Abtrennung des Sulfons kann beispielsweise durch Filtration oder durch Destillation des 4-Nitrotoluols erfolgen.

Das erfindungsgemäße Verfahren kann diskontinuierlich oder kontinuierlich durchgeführt werden. Bei diskontinuierlicher Arbeitsweise kann beispielsweise ein Rührkessel verwendet werden.

Bei kontinuierlicher Arbeitsweise kann die Reaktion beispielweise in einer Rührkesselkaskade, in einem Reaktionsrohr, in einem Dünnschichtreaktor oder in einem Schlaufenreaktor, gegebenenfalls mit Nachreaktoren, durchgeführt werden.

Das gasförmige SO$_3$ wird erfindungsgemäß unverdünnt in das Reaktionsgemisch eingeleitet. Technische Verunreinigungen des SO$_3$, beispielsweise mit SO$_2$, bleiben für das erfindungsgemäße Verfahren ohne negative Auswirkungen.

In einer bevorzugten Ausführungsform wird das erfindungsgemäße Verfahren kontinuierlich durchgefürht, indem beispielsweise gasförmiges, unverdüuntes SO$_3$ gleichzeitig mit geschmolzenem 4-Nitrotoluol in einen Reaktor durch ein Einleitsystem in das Reaktionsgemisch geleitet und dieses in ein Verweilzeitsystem, wie beispielsweise eine Rührkesselkaskade, übergeführt wird. Aus dem Hauptreaktor oder gegebenenfalls dem Verweilzeitsystem fließt das heiße Sulfiergemisch in eine Hydrolyseapparatur, beispielsweise eine Rührapparatur, der gleichzeitig die zur Hydrolyse und Herstellung der 4-Nitrotoluol-2-sulfonsäure-Lösung der gewünschten Konzentration notwendige Wassermenge kontinuierlich zugeführt wird.

Die Hydrolyse-Rohlösung oder Mischung gelangt durch einen Überlauf gegebenenfalls in ein Verweilzeitsystem, wie beispielsweise einen weiteren Rührkessel, von wo aus sie, gegebenenfalls nach Abtrennung von ungelöstem 4-Nitrotoluol und 5,5'-Dinitro-2,2'-ditolylsulfon, in eine Extraktionsapparatur eingespeist wird.

In einer anderen Ausführungsform kann das Sulfon vor der Extraktion durch Filtration abgetrennt werden. Zur Extraktion wird beispielsweise Toluol im Gegenstrom bei etwa 30 bis 70°C im Gewichtsverhältnis Rohlösung : Toluol von etwa 10 : 1 bis etwa 20 : 1 eingespeist.

Nach der Abtrennung der organischen Phase kann das organische Lösungsmittel redestilliert und erneut in die Extraktionsapparatur eingespeist werden. Gegebenenfalls wird das abgetrennte und/oder extrahierte 4-Nitrototuol redestilliert und in den Hauptreaktor zurückgeführt. Das Sulfon kann in kristalliner Formals dem Destillationssumpf gewonnen und einer anderen Verwendung zugeführt werden.

Die nach dem erfindugsgemäßen Verfahren hergesteilte 4-Nitrotoluol-2-sulfonsäure-Lösung kann zur Herstellung sehr reiner, kristalliner 4-Nitrotoluol-2-sulfonsäure verwendet werden.

Außerdem kann 4-Nitrotoluol-2-sulfonsäure zur Herstellung von 4,4'-Dinitrostilben-2,2'-disulfonsäure verwendet werden, wobei z.B. eine ca. 30 bis 50%ige wäßrige Lösung von 4-Nitrotoluol-2-sulfonsäure mit Oxidationsmitteln, beispielsweise mit Luftsauerstoff, umgesetzt wird. Die 4,4'-Dinitrostilben-2-2'-disulfonsäure kann durch Reduktion zur 4,4'-Diaminostilben-2,2'-disulfonsäure weiterverarbeitet werden (siehe Ber. 30, 31000) und ist ein wichtiges Zwischenprodukt für die Herstellung von optischen Aufhellern (A. Doriars, C. W. Schellhammer, J. Schroeder, Angew. Chem. 87, 693 (1975)).

Bei der Umsetzung von 4-Nitrotoluol-2-sulfonsäure zu 4,4'-Dinitrostilben-2,2'-di-sulfonsäure werden besonders hohe Qualitätsanforderungen an die wäßrige Lösung der 4-Nitrotoluol-2-sulfonsäure gestellt (vgl. DE–OS 2 419 455). Die geforderte Qualität wird, wie zuvor beschrieben, ohne weiteres durch das erfindungsgemäße Verfahren erreicht.

Die nachfolgen aufgeführten Beispiele sollen das erfindungsgemäße Verfahren näher erläutern ohne es jedoch auf dieses Beispiele zu beschränken.

Beispiel 1

In die auf 100°C erwärmte Schmelze von 137 g (1 Mol) p-Nitrotoluol werden im Laufe von 40 Minuten 75,6 g (0,94 Mol) gasförmiges Schwefeltrioxid eingeleitet. Die während des Einleitens durch Kühlung auf 100°C gehaltene Schmelze, aus der sich nach ca. 30 Minuten, vom Beginn des Einleitens an gerechnet, Kristalle abscheiden, wird 1 Stunde bei gleicher Temperatur gerührt. Die Analyse einer Probe nach Hydrolyse in Wasser (30 Minuten, 90°C) weist im Reaktionsgemisch (212,6 g) einen Gehalt auf von :

91,3% p-Nitrotoluolsulfonsäure-(2),

6,5% p-Nitrotoluol und

2,1% Schwefelsäure, d.h.

94,6% des Schwefeltrioxids sind umgesetzt zu p-Nitroluolsulfonsäure.

**Beispiel 2**

In eine 0,3 L Rührapparatur werden gleichzeitig 296 g/h gasförmiges $SO_3$ und 533,3 g/h flüssiges 4-Nitrotoluol kontinuierlich durch Einleitrohre eindosiert, wobei die Innentemperatur auf 110°C gehalten wurde. Die gut durchmischte, dünnflüssige Sulfiermasse fließt durch einen Überlauf in eine 2 l Rührapparatur, die gleichfalls auf 110°C temperiert wird. Aus diesem Verweilzeitgefäß fließt die Sulfiermasse durch einen Überlauf in einen 3 l Rührkolben, in den gleichzeitig ca. 1450 g/h Wasser eingespeist werden; die Temperatur wird auf 95°C eingestellt. Durch einen Überlauf gelangt die 4-Nitrotoluol-2-sulfonsäure-Rohlösung nach Filtration in einen Vorratsbehälter.

Durch Extraktion (5 mal mit je 150 ml Toluol) erhält man nach Andestillieren zur Entfernung des Rest-Toluols ca. 2220 g/h einer 4-Nitrotoluol-2-sulfonsäure-Reinlösung (Gardner Färbestandard : Farbzahl 6) der Zusammensetzung :

758,7 g  4-Nitrotoluol-2-sulfonsäure

22,8 g  $H_2SO_4$

≤ 5 mg Summe aller organischen Verunreinigungen

**Beispiel 3**

In der gleichen Apparatur, wie unter Beispiel 2 beschrieben werden bei 120°C 325 g/h $SO_3$ (gasförmiges) und 533 g/h 4-Nitrotoluol eingespeist. Das Verweilzeitgefäß wird ebenfalls auf 120°C gehalten. Die Hydrolyse erfolgt bei 95°C mit ca. 1560 ml $H_2O$/h. Nach Filtration des Suflons (6,7 g/h) und Extraktion (5 mal mit je ca. 200 ml Toluol) bei 60°C und Abdestillieren des Rest-Toluols erhält man 2360 g/h einer 4-Nitrotoluol-2-sulfonsäure-Reinlösung der Zusammensetzung :

816,2 g  4-Nitrotoluol-2-sulfonsäure

27,5 g  $H_2SO_4$

⟨ 5 mg Summe aller organischen Verunreinigungen

Farbzahl 6 (Gardner Färbestandard).

**Ansprüche**

1. Varfahren zur Herstellung von 4-Nitrotoluol-2-sulfonsäure durch Umsetzung von geschmolzenem 4-Nitrotoluol mit gasförmigen Schwelfeltrioxid bei erhöhter Temperatur, Nachreaktion des Reaktionsgemisches und anschließendes Zusammengeben mit Wasser, dadurch gekennzeichnet, daß man in das geschmolzene 4-Nitrotoluol bei einer Temperatur im Bereich von 90 bis 150°C unverdüntes gasförmiges Schwefeltrioxid, das technische Verunreinigungen enthalten kann, bei Drücken oberhalb des Dampfdruckes des 4-Nitrotoluols im Molverhältnis von Schwefeltrioxid zu 4-Nitrotoluol von 0,7 : 1 bis 1,3 : 1 einleitet und das Sulfiergemisch nach Erreichen eines Umsatzes von mindestens 85% der im Unterschuß eingesetzten Reaktionskomponente mit Wasser zusammengibt.

2. Verfahren nach Anspruch 1, dadurch gekennzeichnet, daß das Reaktionsgemisch durch Extraktion mit mit Wasser nicht beliebig mischbaren organischen Lösungsmitteln gereinigt wird.

3. Verfahren nach Anspruch 2, dadurch gekennzeichnet, daß man als organische Lösungsmittel chlorierte aliphatische Kohlenwasserstoffe mit 1 bis 5 Kholenstoffatomen verwendet.

4. Verfahren nach Anspruch 2, dadurch gekennzeichnet, daß man als organische Lösungsmittel aromatische Kohlenwasserstoffe mit 6 bis 18 Kohlenstoffatomen verwendet.

5. Verfahren nach Anspruch 2, dadurch gekennzeichnet, daß man als organische Lösungsmittel Methylenchlorid und/oder Toluol verwendet.

**Claims**

1. Process for the preparation of 4-nitrotoluene-2-sulphonic acid by reaction of molten 4-nitrotoluene with gaseous sulphur trioxide at elevated temperature, postreaction of the reaction mixture and subsequent combination with water, characterised in that undiluted gaseous sulphur trioxide, which may contain technical impurities, is passed into the molten 4-nitrotoluene at a temperature in the range from 90 to 150°C, at pressures above the vapour pressure of the 4-nitrotoluene in a molar ratio of sulphur trioxide to 4-nitrotoluene of 0.7 : 1 to 1.3 : 1 and the sulphonation mixture is combined with water after achieving a conversion of at least 85% of the reaction component employed in the sub-equivalent amount.

2. Process according to Claim 1, characterised in that the reaction mixture is purified by extraction with completely water-immiscible organic solvents.

3. Process according to Claim 2, characterised in that chlorinated aliphatic hydrocarbons having 1 to 5 carbon atoms are used as organic solvents.

4. Process according to Claim 2, characterised in that aromatic hydrocarbons having 6 to 18 carbon atoms are used as organic solvents.

5. Process according to Claim 2, characterised in that methylene chloride and/or toluene are used as organic solvents.

## Revendications

1. Procédé de préparation de l'acide 4-nitrotoluène-2-sulfonique par réaction du 4-nitrotoluène fondu avec l'anhydride sulfurique gazeux à chaud, réaction complémentaire du mélange de réaction suivie d'un contact avec l'eau, caractérisé en ce que l'on envoie dans le 4-nitrotoluène fondu à une température dans l'intervalle de 90 à 150°C de l'anhydride sulfurique gazeux non dilué, qui peut contenir des impuretés industrielles, à des pressions supérieures à la pression de vapeur du 4-nitrotoluène dans un rapport molaire anhydride sulfurique/4-nitrotoluène de 0,7 : 1 à 1,3 : 1 et on met le mélange de sulfonation en contact avec de l'eau lorsqu'on a atteint un taux de conversion d'au moins 85 % pour le composant de la réaction mis en oeuvre en défaut.

2. Procédé selon la revendication 1, caractérise en ce que le mélange de réaction est purifié par extraction à l'aide de solvants organiques non miscibles en toutes proportions à l'eau.

3. Procédé selon la revendication 2, caractérisé en ce que l'on utilise en tant que solvants organiques des hydrocarbures aliphatiques chlorés en $C_1$-$C_5$.

4. Procédé selon la revendication 2, caractérisé en ce que l'on utilise en tant que solvants organiques des hydrocarbures aromatiques en $C_6$-$C_{18}$.

5. Procédé selon la revendication 2, caractérisé en ce que l'on utilise en tant que solvants organiques le chlorure de méthylène et/ou le toluène.